(19) Europäisches Patentamt

European Patent Office

Office européen des brevets

(11) **EP 0 823 627 A1**

(12) **EUROPEAN PATENT APPLICATION**

(43) Date of publication:
**11.02.1998 Bulletin 1998/07**

(51) Int. Cl.$^6$: **G01N 21/41**

(21) Application number: **97109874.4**

(22) Date of filing: **17.06.1997**

(84) Designated Contracting States:
**AT BE CH DE DK ES FI FR GB GR IE IT LI LU MC NL PT SE**

(30) Priority: **09.08.1996 SE 9602960**

(71) Applicant: **Siemens-Elema AB**
**171 95 Solna 1 (SE)**

(72) Inventors:
• **Krahbichler, Erik**
  **170 71 Solna (SE)**
• **Pessala, Tom**
  **168 32 Bromma (SE)**

(54) **Device for identifying liquid anaesthetics**

(57)    The present invention relates to a device (64), intended for use in anaesthetic machines, for identifying at least one liquid anaesthetic (72) in the anaesthetic machine. The device (64) comprises a measuring unit (74) for determining at least one parameter related to the refractive index of the liquid anaesthetic (72), a means (92, 94) for determining the temperature of the liquid anaesthetic (72) and an analysis unit (80) for identifying the anaesthetic (72) from the determined parameter.

FIG. 3

## Description

The present invention relates to a device, intended for use in anaesthetic machines, for identifying at least one liquid anaesthetic in the anaesthetic machine.

In principle, narcosis or anaesthesia involves the induction of a dormant state in a patient in which she/he is incapable of feeling any pain. The patient is usually administered a mixture of oxygen, nitrous oxide, an anaesthetic agent and (possibly) air from a respiratory circuit in an anaesthetic machine. The most common anaesthetic agents are halothane, desflurane, enflurane, isoflurane and sevoflurane. The anaesthetic agent is normally in liquid form in an anaesthetic vaporiser attached to the anaesthetic machine, and a desired amount of liquid anaesthetic is vaporised and sent to the respiratory circuit during anaesthesia.

The effect of different anaesthetic agents on the patient, which are administered to the patient in varying concentrations, varies. The side-effects of the different anaesthetic agents also varies. So anaesthetic machines are available which can be equipped with a plurality of anaesthetic vaporisers so as to give the anaesthetist a direct option of selecting the anaesthetic agent she/he deems best for the patient, with no need to mount or detach different anaesthetic vaporisers. In addition, the anaesthetist is also able to switch to a different anaesthetic agent during ongoing surgery. This occurs in operations on e.g. infants or in protracted surgery.

But the patient should never be given a mixture of different anaesthetic agents, since the effect of the mixture is hard to predict and largely unknown. That is why anaesthetic machines are normally devised so they are only able to administer one anaesthetic agent at a time to the patient. However, the mixing of a plurality of anaesthetic gases is still a risk, even with anaesthetic machines with only one anaesthetic vaporiser, viz. if different anaesthetic agents became intermixed when anaesthetic agent is added to the active anaesthetic vaporiser.

To minimise the risk of errors in the induction of anaesthesia, it would be advantageous if the anaesthetic system could automatically identify the anaesthetic agent administered to the patient. The anaesthetic machine could be devised to issue an alarm if an error occurred.

As noted above, the various known anaesthetic agents are administered to the patient in different concentrations. If an erroneous concentration is set for a particular anaesthetic agent, the patient could be subjected to needless risk, entailing both overdosing and underdosing of the anaesthetic agent. Erroneous concentration will therefore most likely occur when wrong anaesthetic agent is administered, since different concentrations are used for the different anaesthetic agents.

Even these risks would be greatly reduced if the anaesthetic agent could be identified before it is supplied to the patient.

In the anaesthesia art, identification of anaesthetic agents by optical methods, i.e. by absorption spectrophotometry, is known. Since a plurality of anaesthetic agents have similar absorption spectra, absorption must therefore be measured at a plurality of wavelengths to permit reliable identification of a specific anaesthetic agent. This makes it necessary to use extensive measurement equipment in identifying an anaesthetic agent. Optical measurement is generally performed on the gas mixture supplied to the patient, i.e. the anaesthetic agent is present in gaseous form.

Another known way of identifying anaesthetics in gaseous form is based on the use of two different measurement methods for determining the concentration of the anaesthetic agent and of a combination of these methods so the identity of the anaesthetic agent can also be established. For example, an optical method, in which measurement of absorption is made at a specific wavelength for the anaesthetic agent, and a method using an oscillating crystal, coated with a layer of oil or grease which adsorbs and desorbs anaesthetic gas molecules, can be used. The crystal's oscillation frequency changes to varying degrees, depending on the molecular weight and concentration of the anaesthetic gas. When a combination of these two measurement methods is employed, a unique signal is obtained for every known anaesthetic agent. It should be noted that measurement employing changes in the oscillation frequency of a crystal is not in itself sufficient to permit identification of anaesthetic agents, since the frequency change is relatively the same for the different anaesthetic agents in the concentrations at which they are normally used. One such device is described in US-A 5 272 907.

The present invention is directed to solving the aforementioned problem. This is achieved with a device, intended for use in anaesthetic machines, for identifying at least one liquid anaesthetic in the anaesthetic machine according to claim 1. The device according to the present invention comprises a measuring unit for determining at least one parameter related to the refractive index of the liquid anaesthetic and means for determining the temperature of the liquid anaesthetic. The device further comprises an analysis unit for identifying the anaesthetic agent from the determined parameter an.

One advantage of the device according to the present invention is that the analysis unit is able to determine, from the established parameters, whether a mixture of at least two anaesthetic agents is present. This further enhances patient safety in anaesthesia. Preferably, the analysis unit is adapted to also determine which liquid anaesthetics that are mixed and the concentration of each liquid anaesthetic.

A first possibility to obtain a parameter is achieved in accordance with the invention in that a light-emitting means is arranged so a light beam emitted by it strikes the surface of the liquid anaesthetic at a specific angle of incidence. The device according to the present invention also comprises a position detector which is arranged to indicate any deflection of the light beam in the liquid. This deflection constitutes the parameter and depends on the liquid anaesthetic's refractive index and level.

The level can be made into a constant by selecting the appropriate arrangement, e. g. by using a small container which receives a precise amount of liquid from the vaporiser. Such a test can be made at onset of the machine and each time the vaporiser is filled with liquid anaesthetic. When the level is constant, the identity of the liquid anaesthetic can be determined based on temperature and deflection (refractive index).

In conjunction herewith, it would be advantageous if a measuring rod were arranged on the position detector to indicate the identity of the anaesthetic agent when the light beam strikes the position detector. If the light beam shines between two different "identity" positions, there is a mixture of different anaesthetics.

A second possibility to obtain a parameter is achieved when the measuring unit also comprises an optical fibre which has one end by the light-emitting means, the other end arranged by a photosensitive means and a curved section immersed in the liquid anaesthetic. The measuring unit is also arranged to determine the amount of light which leaks out of the curved section of the optical fibre as the parameter.

Although similar devices are known (see e. g. US-A-4,187,025) for determining refractive index or temperature of a known liquid, or discrete levels of a liquid in a container, they have not been utilised, nor suggested for use in determining the identity of anaesthetics in anaesthetic machines.

According to a first aspect of the invention, the part of the optical fibre immersed in the liquid anaesthetic has straight sections in addition to the curved section. In particular, the straight sections should pass the surface at a right angle. Hereby, a minimum of losses of light from the fibre is obtained as it passes from one surrounding refractive index (e. g. air) to another (the liquid anaesthetic).

In conjunction herewith, it would be advantageous if the measuring unit also comprised a second optical fibre, not immersed in the liquid anaesthetic, of the same length as the first fibre. This second fibre is intended to supply a reference value.

According to a second aspect of the invention, the measuring unit also comprises n similar optical fibres arranged at different levels in the liquid in order to determine the level of the liquid in discrete steps.

In conjunction herewith, it would be advantageous if at least one optical fibre had a plurality of curved sections on different levels in the liquid in order to determine the level of the liquid.

An additional advantage in conjunction herewith would be if the analysis unit issued an alarm when the level of the liquid dropped below the curved section of the fibre.

An alternative possibility to obtain simultaneous level measurements with the parameter determination, is achieved in accordance with the invention in that the device also comprises two electrode means for contacting any liquid anaesthetic present and measuring the capacitance C of liquid between the electrode means, whereby the liquid's C varies with and, thus, supplies a measure of the level of the liquid.

According to a first aspect of the invention according to the third possibility, the immersed part of the optical fibre has two essentially parallel sections, and the two electrode means are arranged along these essentially parallel sections.

In conjunction herewith, it would be advantageous if the two electrode means consisted of a metallized surface area on the optical fibre's parallel sections.

Another advantageous alternative in conjunction therewith would be if the two electrode means consisted of metal wire wound around the optical fibre's parallel sections.

According to a second aspect of the invention according to the third possibility, the first electrode means consists of a metallized surface area on the optical fibre, and the second electrode means consists of a metallic deposit on the inside of a container for the liquid anaesthetic.

The invention will now be explained below in greater detail by the following description of preferred embodiments of same, referring to the attached drawings.

FIG. 1 shows an anaesthetic machine;
FIG. 2 shows a first embodiment of the device according to the invention;
FIG. 3 shows a second embodiment of the device;
FIG. 4 shows a third embodiment of the device;
FIG. 5 shows a fourth embodiment of the device;
FIG. 6 shows a fifth embodiment of the device;
FIG. 7 shows a sixth embodiment of the device;
FIG. 8 shows a seventh embodiment of the device;
FIG. 9 shows an eighth embodiment of the device;
FIG. 10 shows a ninth embodiment of the device;
FIG. 11 shows a tenth embodiment of the device;
FIG. 12 shows an eleventh embodiment of the device.

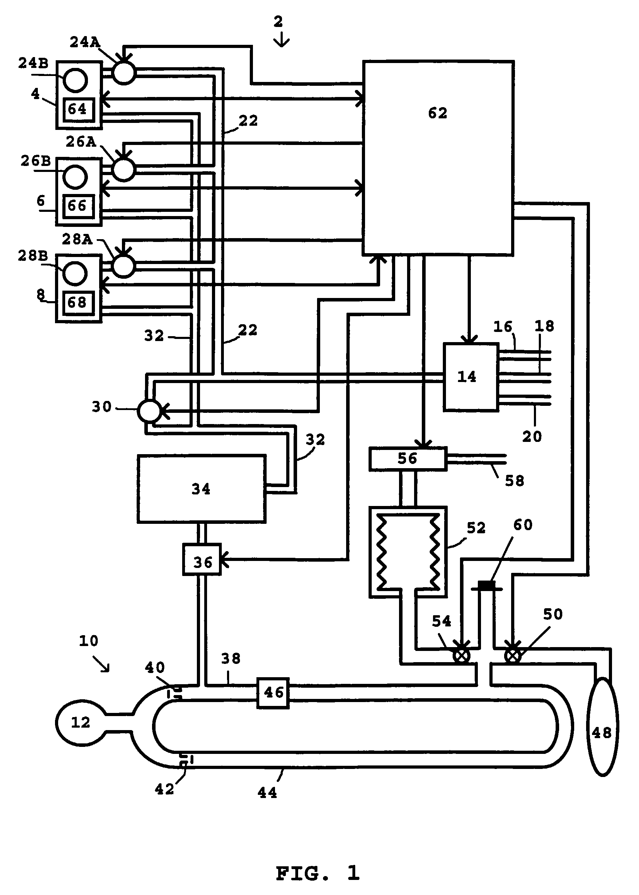

FIG. 1 shows an anaesthetic machine 2 to which a first anaesthetic vaporiser 4, a second anaesthetic vaporiser 6 and a third anaesthetic vaporiser 8 are attached for selective supply of an anaesthetic agent to a respiratory circuit 10. The respiratory circuit 10 then delivers a respiratory gas containing the anaesthetic gas to the patient 12.

The respiratory gas is fed to the anaesthetic machine 2 via a gas mixer 14. Gas can be carried to the gas mixer 14 via a first gas connector 16, a second gas connector 18 and a third gas connector 20. The supplied gases can be air, nitrous oxide and oxygen. If the anaesthetic machine 2 is only to be supplied with oxygen and nitrous oxide, the third gas connector 20 can either be turned off or also used for supplying oxygen. The supply of oxygen via two separate gas connectors 18, 20 increases patient safety. Arriving gases are mixed in the gas mixer 14 in variable proportions to form a respiratory gas at a pre-selected pressure, whereupon a pre-selected flow of the mixed respiratory gas is carried through a first gas line 22 to the anaesthetic vaporisers 4, 6, 8.

A first stop valve 24A is arranged by the first anaesthetic vaporiser 4. The first stop valve 24A, which is normally closed, prevents gas from the first gas line 22 from passing through the first anaesthetic vaporiser 4. When an operator activates a first setting knob 24B on the first anaesthetic vaporiser 4, the first stop valve 24A opens, and liquid anaesthetic in the first anaesthetic vaporiser 4 is vaporised so the concentration set for anaesthetic in the respiratory gas is achieved. In the corresponding manner, a second stop valve 26A is arranged by the second anaesthetic vaporiser 6, and a third stop valve 28A is arranged by the third anaesthetic vaporiser 8. The second stop valve 26A opens when a second setting knob 26B is activated, and the third stop valve 28A opens when a third setting knob at the third anaesthetic vaporiser 8 is activated. The three stop valves 24A, 26A, 28A are regulated so only one can be activated at any given time.

A fourth stop valve 30 is arranged by the first gas line 22 to pass a flow of gas which has not passed through any of the anaesthetic vaporisers, 4, 6, 8.

The anaesthetic machine 2 is devised so the fourth stop valve 30 automatically opens if the first stop valve 24A, the second stop valve 26A and the third stop valve 28A are closed. This ensures that the patient has a supply of respiratory gas at all times.

Respiratory gas from the gas mixer 14 is then carried, with or without anaesthetic gas, through a second gas line 32 to a gas reservoir 34. The respiratory gas is additionally mixed in the gas reservoir 34 to ensure that vaporised anaesthetic agent is mixed with the respiratory gas to greatest possible degree before the latter is sent on the respiratory circuit 10.

In this instance, the respiratory circuit 10 consists of a recirculating respiratory circuit in which the patient 12 rebreathes a greater or lesser part of the gas in the respiratory circuit 10. Here, the mixed respiratory gas in the gas reservoir 34 can suitably be designated "fresh gas" for the respiratory circuit 10. Fresh gas is supplied to the respiratory circuit 10 to compensate for the loss or leakage of gas from the respiratory circuit 10, caused by e.g. the uptake of oxygen and anaesthetic gas in the patient's 12 body and leakage in the entire circuit system (the respiratory circuit 10 and the patient 12).

The supply of fresh gas to the respiratory circuit 10 is regulated by a fresh gas regulator 36. Fresh gas is sent to an inspiratory line 38 in the respiratory circuit 10 and delivered to the patient 12 through a first check valve 40. Gas expired by the patient 12 is carried through a second check valve 42 and an expiratory line 44. A carbon dioxide absorber 46 is also arranged in the respiratory circuit 10.

Two possible drive systems are indicated for respiratory gas in the respiratory circuit 10. The first consists of a manually squeezed breathing bag 48 which can be connected to the respiratory circuit 10 via a valve 50. When the breathing bag 48 is attached to the respiratory circuit 10, a physician can manually squeeze the breathing bag 48 in order to control the patient 12's inspirations and expirations. Alternately, gas in the respiratory circuit 10 can be mechanically acted upon by a respiratory control unit 52, consisting of a bag inside a container which can be connected to the respiratory circuit 10 via a valve 54. The respiratory control unit 52 is regulated by a drive unit 56, employing compressed air from a fourth gas connector 58, which can send a drive gas to the respiratory control unit 52 and evacuate drive gas from same. Surplus gas in the respiratory circuit 10 is evacuated via a relief valve 60.

The anaesthetic machine 2 is controlled and monitored by a control device 62. The control device 62 regulates the operation of the gas mixer 14, drive unit 56, fresh gas regulator 36, stop valves 24A, 26A, 28A, 30 and anaesthetic vaporisers 4, 6, 8. The control device 62 also receives information on settings for functions, such as breathing rate, desired tidal volume, respiratory gas composition etc., made by staff. Other functions and tasks performed by the control device 56 will be apparent from the following.

In order to e.g. identify the anaesthetic agents connected to the anaesthetic machine 2, each anaesthetic vaporiser 4, 6, 8 comprises a device 64, 66, 68 for identifying the anaesthetic agent in the respective anaesthetic vaporiser 4, 6, 8. Information ascertained here on the anaesthetic agent in each anaesthetic vaporiser 4, 6, 8 is sent to the control device 62.

A plurality of embodiments of the device 64, 66, 68 are possible. Eleven embodiments of the device 64, 66, 68 are described below.

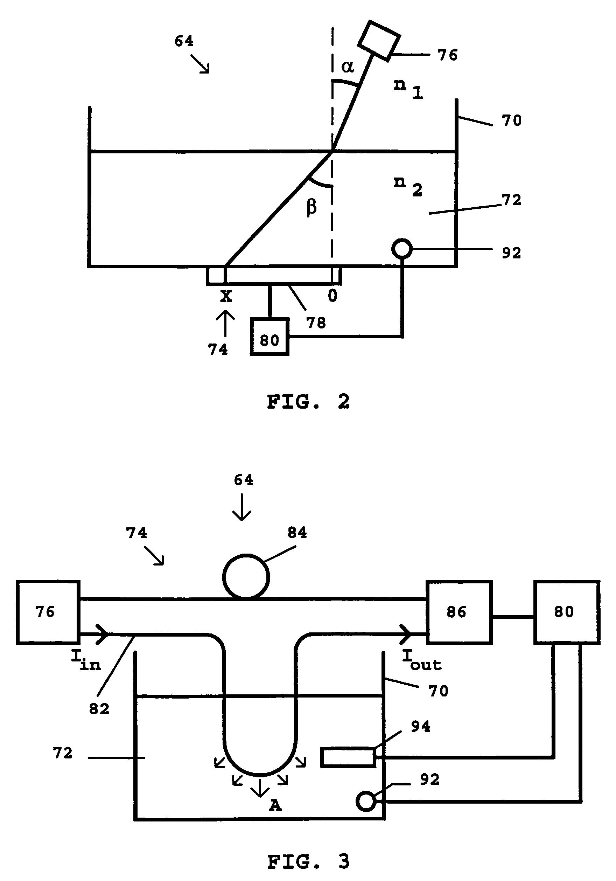

FIG. 2 shows a first embodiment of the device 64 (the designations 66 and 68 will henceforth be omitted for the sake of simplicity) according to the present invention. The device 64 can, but does not need to, include a container 70 for holding a liquid anaesthetic 72. The device 64 also comprises a measuring unit 74 and an analysis unit 80. The measuring unit 74 comprises a light-emitting means 76 and a position detector 78. The light-emitting means 76 emits a light beam which strikes the surface of the liquid anaesthetic 72 at a specific angle of incidence $\alpha$. Since the anaesthetic agent 72 has a refractive index $n_2$ which differs from the refractive index $n_1$ of the medium (e.g. air) above the anaesthetic agent 72, the light beam is refracted or deflected in the liquid at an angle of refraction $\beta$. The degree to which the light is refracted depends on the liquid anaesthetic's 72 refractive index $n_2$. This relationship can be expressed with the law of refraction which states that:

$$n_1 \sin \alpha = n_2 \sin \beta$$

The deflected light beam strikes the position detector 78 at x. If $n_1$, $\alpha$, the level of liquid in the container 70 and the temperature of the liquid are known, the refractive index $n_2$ of the liquid anaesthetic 72 can be determined with the aid of x.

Temperature is measured by a thermometer 92, which is connected to the analysis unit 80 for providing measurement of the temperature to same.

The level can also be measured (not shown) but the container 70 could be constructed so that a defined level of liquid is maintained in the measurement (analysis) area.

The analysis unit 80, connected to the position detector 72 and the thermometer 92 performs this determination of $n_2$ which is used for establishing the liquid anaesthetic's identity. The position detector 78 can also be equipped with a measuring rod (not shown) which is marked for directly indicating the identity of the anaesthetic agent 72 at the position the light beam strikes the position detector 78. (In the latter case, a control of the temperature and level of the liquid to preselected constants is necessary.)

In another version, a ruler can serve as the position detector 78, and a window is arranged so the ruler can be observed. A light dot will be seen when the light beam strikes the ruler, and the identity of the anaesthetic agent can be read on the ruler (which accordingly serves as an analysis unit).

The analysis unit 80 is also capable of determining whether a mixture of at least two anaesthetic liquids is present. Basically, the light beam will then light a position on the rod 78 which should not be lit.

It should be noted that numerous variations of this arrangement are possible. For instance, the light source 76 can be immersed in the liquid and the rod 78 placed above the surface. An liquid - glass interface can be used instead of a liquid - air interface. The container 70 can have a prismatic shape, and the light can be transmitted through the prism for determining the deflection (refractive index of the liquid). The rod 78 can be made to fluoresce where the transmitted light hits it, thereby illuminating the spot of interest.

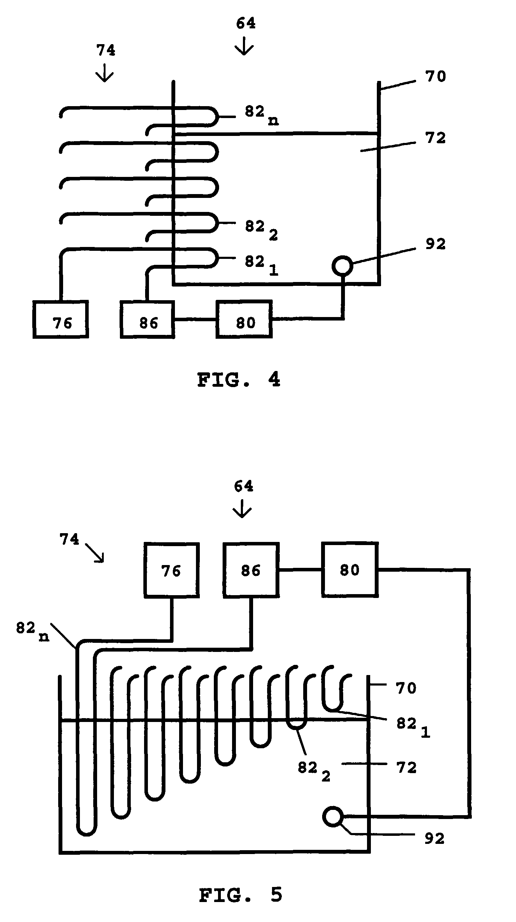

FIG. 3 shows a second embodiment of the device 64 according to the present invention. The device 64 comprises a container 70 (which does not need to be included in the device 64) for holding an anaesthetic agent 72 in liquid form. The device 64 also comprises a measuring unit 74, a thermometer 92 and an analysis unit 80. In the alternative, a thermostat 94 can be used for controlling the temperature of the liquid - maintaining it at a selected level. Such control could be exercised from the analysis unit 80.

The measuring unit 74 comprises a light-emitting means 76, a first optical fibre 82, a second optical fibre 84 and a photosensitive means 86. The analysis unit 80 is connected to the photosensitive means 86 and to the thermometer 92. The first optical fibre 82 has a first end coupled the light-emitting means 76 for receiving light ($I_{in}$) and its other end coupled to the photosensitive means 86 for emitting transmitted light ($I_{out}$). The first optical fibre 82 is also partially immersed in the liquid anaesthetic 72.

The immersed part consists of at least one curved section. In the illustrated device 64, the immersed part of the first optical fibre 82 also comprises two essentially parallel sections. In particular, the parallel sections should pass the liquid surface at a right angle in order to avoid unwonted leakage of light.

The second optical fibre 84 has the same length as the first optical fibre 82 and has a first end arranged by the light-emitting means 76 and its other end arranged by the photosensitive means 86. The second optical fibre 84 is intended to supply a reference value. When an optical fibre is curved, as the first optical fibre 82 is, light leaks out of the curved section, as designated with the arrows A. No light leaks out of the optical fibre's straight sections. The amount of light leaking out of the curved section depends on the refractive index of the liquid anaesthetic 72, in addition to the fibre's radius of curvature and diameter (however, these latter units are known and taken into consideration in the algorithms for determining the identity of the liquid anaesthetic 72). The refractive index can therefore be determined based on the $I_{in}/I_{out}$ ratio.

The measuring unit 74 is thus devised to determine the amount of light leaking out of the curved section of the first optical fibre 82 as the parameter related to refractive index. As the anaesthetic agent is unknown, the measuring unit 80 will, with the aid of this parameter (amount of leaked light A) and the reference value, identify the anaesthetic agent, since the amount of light received in relation to the amount emitted is a measure of the refractive index of the liquid. All other parameters (e. g. temperature) are known.

Here also, numerous variations are possible. Some will be described below, others are obvious. For instance, the curved part of the fibre 82 can have a plurality of different shapes for enhancing the measurement to the ruling conditions, i. e. the interval of refractive indices (anaesthetic agents), the material of the fibre, the frequency or frequencies of light that is used, etc.

FIG. 4 shows a third embodiment of the device 64 according to the present invention. The device 64 comprises a container 70 (which does not need to be included in the device 64) for holding an anaesthetic agent 72 in liquid form. The device 64 also comprises a measuring unit 74, a thermometer 92 and an analysis unit 80. As in the above embodiment, the thermometer 92 can be replaced with a thermostat for maintaining a pre-selected temperature of the liquid anaesthetic. The measuring unit 74 comprises a light-emitting means 76, n optical fibres $82_1$, $82_2$, ..., $82_n$ and a photosensitive means 86. Each optical fibre has a first end arranged by the light-emitting means 76 and its other end arranged by the photosensitive means 86. All the fibres $82_1$, $82_2$, ..., $82_n$ have a curved section, and the fibres are arranged so their curved sections are arrayed at different levels in the container 70.

Since the amount of light leaking out of the curved section of the optical fibre depends on the refractive index of the medium, the device 64 can determine whether or not a specific fibre's curved section is in the liquid anaesthetic 72. The number of fibres arranged in the liquid anaesthetic 72 designates the level of the liquid in discrete steps. The greater the number of fibres $82_1$, $82_2$, ..., $82_n$ used, the greater the accuracy in determination of the level of the liquid. One advantage of this embodiment is that the identity of the anaesthetic agent can be determined at the same time as the level of the liquid. Also, if there is a heterogeneous mixture of two anaesthetics, this can be detected by the device since the refractive index will vary at different levels (i. e. different fibres $82_i$ will measure different refractive indices. One light-emitting means and one photosensitive means can obviously be utilised for each fibre.

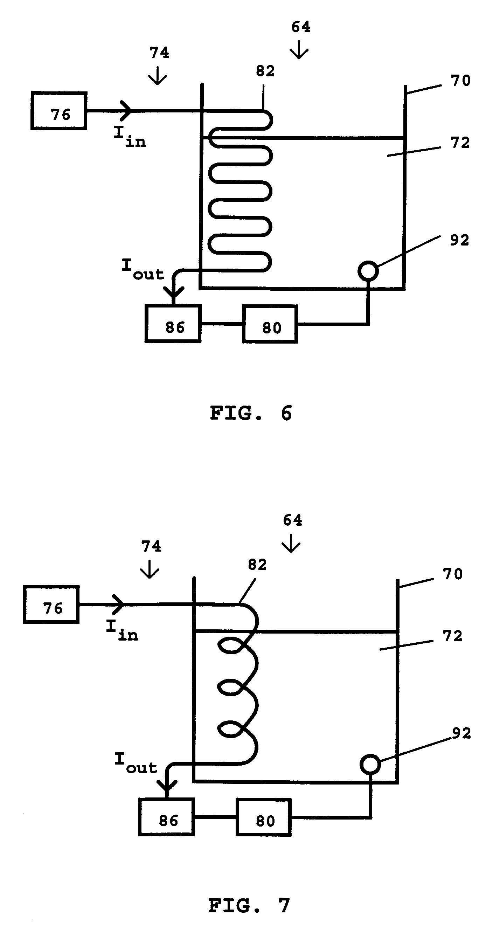

FIG. 5 shows a fourth embodiment of the device 64 according to the present invention. The device 64 comprises a container 70 (which does need to be included in the device 64) for holding an anaesthetic agent 72 in liquid form. The device 64 also comprises a measuring unit 74, a thermometer 92 and an analysis unit 80. As in the above embodiment, the thermometer 92 can be replaced with a thermostat for maintaining a pre-selected temperature of the liquid anaesthetic. The measuring unit 74 comprises a light-emitting means 76, n optical fibres $82_1$, $82_2$, ..., $82_n$ and a photosensitive means 86. Each optical fibre $82_1$, $82_2$, ..., $82_n$ has a first end arranged by the light-emitting means 76 and its other end arranged by the photosensitive means 86. All the fibres $82_1$, $82_2$, ..., $82_n$ have a curved section, and the fibres $82_1$, $82_2$, ..., $82_n$ are arranged so they descend into the container 70 to differing degrees, i.e. they are arranged at different levels. This embodiment is also based on the fact that the amount of light leaking out of the curved section of an optical fibre depends on the refractive index of the medium surrounding the curved section. The number of fibres arranged in the liquid anaesthetic 72 designate the level of the liquid in discrete steps. As in the previous embodiment, the identity of the anaesthetic agent can be determined at the same time as the determination of the level of the liquid. Likewise, determination of mixtures can be made simultaneously. A plurality of light-emitting means and a plurality of photosensitive means can also be provided in this embodiment.

FIG. 6 shows a fifth embodiment of the device 64 according to the present invention. The device 64 comprises a container 70 (which does not need to be included in the device 64) for holding an anaesthetic agent 72 in liquid form. The device 64 also comprises a measuring unit 74, a thermometer 92 and an analysis unit 80. As in the above embodiments the thermometer 92 can be replaced with a thermostat for maintaining a constant temperature. The measuring unit 74 comprises a light-emitting means 76, an optical fibre 82 and a photosensitive means 86. The optical fibre 82 has a first end arranged by the light-emitting means 76 and its other end by the photosensitive means 86. The optical fibre 82 has a plurality of curved sections arranged at different levels in the container 70. The straight sections of the fibre 82 are essentially parallel to the level of any liquid present. This embodiment is also based on the fact that the amount of light leaking out of the curved section of an optical fibre depends on the refractive index of the medium around the curved section. In this case, the identity of the anaesthetic agent and the level of the liquid can be determined simultaneously in discrete steps. The requirements for doing this is that the conditions at onset are known, i. e. that the container 70 is filled up to a specific level with the liquid anaesthetic. Before allowing any part of the liquid to be vaporised, the identity of the anaesthetic agent is determined. This can be made from the $I_{in}/I_{out}$ ratio. After determination of the identity of the anaesthetic agent (or determination of a mixture) liquid may be used for narcosis. As the level drops, the leakage of light changes. This is determined by the analysis unit and the current level can be calculated.

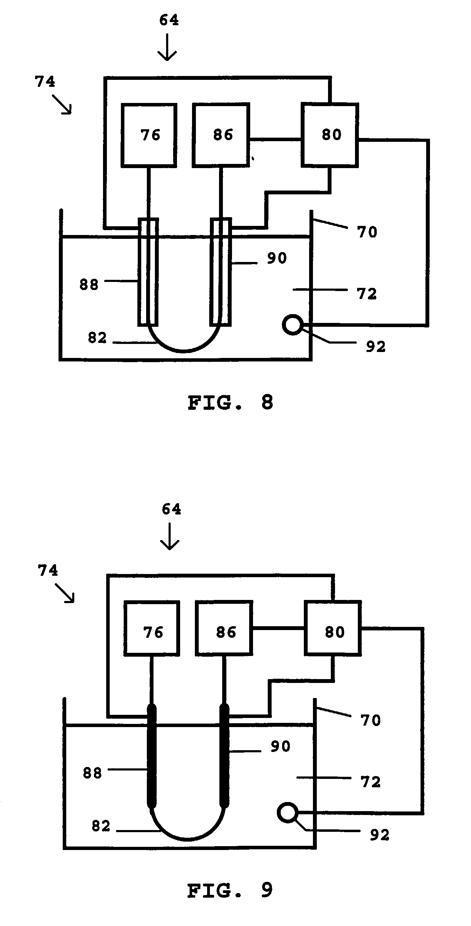

FIG. 7 shows a sixth embodiment of the device 64 according to the present invention. The device 64 comprises a container 70 (which does no need to be included in the device 64) for holding an anaesthetic agent 72 in liquid form. The device 64 also comprises a measuring unit 74, a thermometer 92 and an analysis unit 80. As in the above embodiments, the thermometer 92 can be replaced with a thermostat for maintaining the temperature of the liquid constant. The measuring unit 74 comprises a light-emitting means 76, an optical fibre 82 and a photosensitive means 86. The optical fibre 82 has a first end arranged by the light-emitting means 76 and its other end arranged by the photosensitive means 86. The optical fibre has the shape of a coil arranged in the container 70 so the coil's axis centre line is perpendicular to the level of any liquid present. The signal $I_{out}$ measured by the photosensitive means 86 is a function of the level of liquid in the container 70. Since a coil mainly consists of curved sections, light leaks out of the entire coil. In this case also, the identity of the anaesthetic agent and the level of the liquid can be determined simultaneously in discrete steps. The requirements for doing this is that the conditions at onset are known, i. e. that the container 70 is filled up to a specific level with the liquid anaesthetic. Before allowing any part of the liquid to be vaporised, the identity of the anaesthetic agent is determined. This can be made from the $I_{in}/I_{out}$ ratio. After determination of the identity of the anaesthetic agent (or determination of a mixture) liquid may be used for narcosis. As the level drops, the leakage of light changes. This is determined by the analysis unit and the current level can be calculated.

FIG. 8 shows a seventh embodiment of the device 64 according to the present invention. The device 64 comprises a container 70 (which does no need to be included in the device 64) for holding an anaesthetic agent 72 in liquid form. The device 64 also comprises a measuring unit 74, a thermometer 92 and an analysis unit 80. The thermometer can be replaced with a thermostat for maintaining a constant temperature in the liquid. The measuring unit 74 comprises a light-emitting means 76, an optical fibre 82, a photosensitive means 86, a first electrode means 88 and a second electrode means 90. The optical fibre 82 has a first end arranged by the light-emitting means 76 and its other end arranged by the photosensitive means 86. The optical fibre 82 also has a curved section arranged in the container 70. The photosensitive means 86 and the two electrode means 88, 90 are connected to the analysis unit 80. The two electrode means 88, 90 are arranged along the straight sections of the optical fibre and are arrayed in the container 70 so they come into contact with the liquid anaesthetic 72 when the latter exceeds a specific level in the container 70. The optical fibre 82 is used in the same way as the embodiment according to FIG. 3, i.e. for determining the identity or temperature of the anaesthetic agent 72. With knowledge thereof, the dielectric constant $\varepsilon_r$ can be obtained for the liquid. The electrode means 88, 90 can be used for measuring the capacitance C of the liquid between the electrode means 88, 90. Since the capacitance C of the liquid varies with the level of the liquid, the analysis unit 80 can determine the level of the liquid with the aid of the capacitance C established for the identified liquid.

FIG. 9 shows an eighth embodiment of the device 64 according to the present invention. Identical parts in FIGS. 8 and 9 have been assigned the same reference designations. The difference compared to the device 64 according to FIG. 8 is that the two electrode means 88, 90 consist of a metallized surface area on the optical fibre's 82 straight, parallel sections. Otherwise, it has the same function as the function of the device 64 according to FIG. 8.

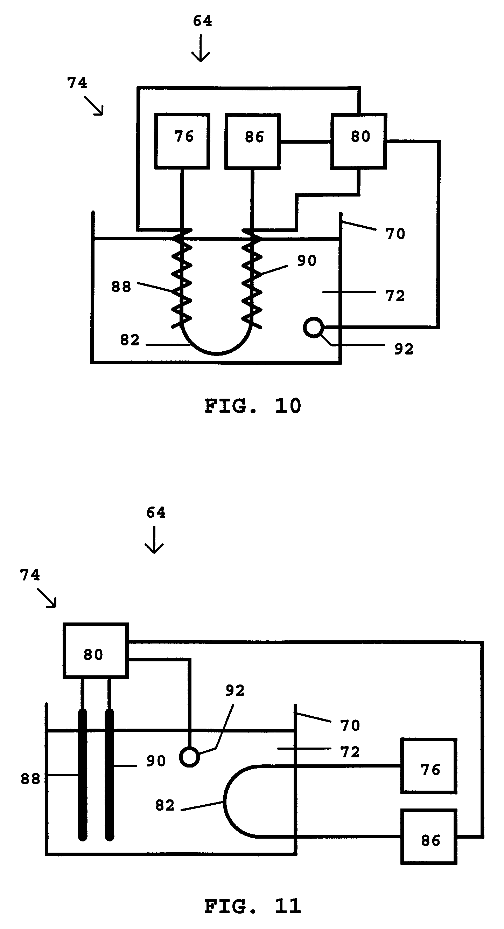

FIG. 10 shows a ninth embodiment of the device 64 according to the present invention. Identical parts in FIGS. 8-10 have been assigned the same reference designations. The difference compared to the devices 64 according to FIGS. 8 and 9 is that the two electrode means 88, 90 consist of metal wire wound around the optical fibre's 82 straight, parallel sections. Otherwise, it has the same function as the function of the devices 64 according to FIGS. 8 and 9.

FIG. 11 shows a tenth embodiment of the device 64 according to the present invention. Identical parts in FIGS. 8-11 have been assigned the same reference designations. The difference compared to the devices 64 according to FIGS. 8-10 is that the two electrode means 88, 90 are physically separate from the optical fibre 82. The two electrode means 88, 90 and the optical fibre 82 do not need to arrayed in the illustrated manner, but the most salient feature of the embodiment according to FIG. 11. Otherwise, it functions the same as the devices 64 according to FIGS. 8-10.

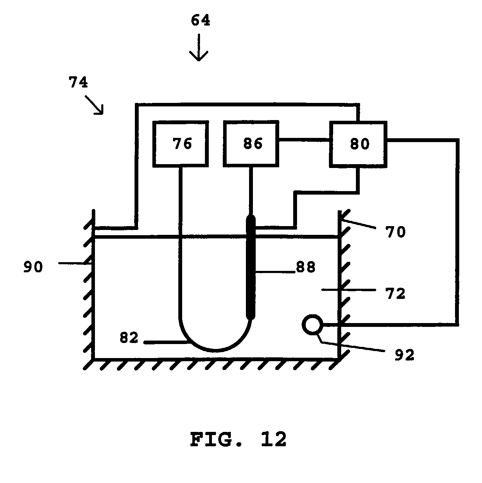

FIG. 12 shows an eleventh embodiment of the device 64 according to the present invention. Identical parts in FIGS. 8-12 have been assigned the same reference designations. The new aspect of this embodiment is that the first electrode means 88 consists of a metallic deposit on the optical fibre 82, and the second electrode means 90 consists of a metallic deposit on the interior of the container 70 for liquid anaesthetic 72. Otherwise, it functions the same way as the devices 64 according to FIGS. 8-11, i.e. the optical fibre 82 is used for determining the identity or temperature of the liquid anaesthetic 72, and the level of liquid is measured with the aid of the varying capacitance C between the two electrode means 88, 90.

The analysis unit 80 in the illustrated devices 64 for measuring the level of liquid can also be equipped with means for issuing an alarm indication, e.g. in the form of an acoustic or visual signal, when a mixture of anaesthetic agents is determined, when the set concentration is outside the normal range for the identified anaesthetic (i. e. there is a risk that the vaporiser is completely filled with wrong anaesthetic or that the anaesthetist has mistakenly activated the wrong vaporiser), and/or when the liquid drops below a specific, pre-defined level in the container 70.

The optical fibre 82 can be made of e.g. fibreglass. Different cladding can used along the fibre 82 for reducing any unnecessary loses of light. It would be sufficient to have only the immersed part exposed for leakage and the rest of the fibre 82 enclosed for guaranteeing reflection of the light in the fibre 82.

The light-emitting means 76 can e.g. consist of a light-emitting diode, a laser, a lamp or any other light generator. The photosensitive means 86 can e.g. consist of a photo diode, photo resistor, or other light detector.

The invention is not limited to the illustrated embodiments. A plurality of variations are possible within the scope of the attached patent claims. E. g. combinations of the enclosed embodiments can be made were appropriate.

**Claims**

1. A device (64), intended for use in anaesthetic machines (2), for identifying at least one liquid anaesthetic (72) in the anaesthetic machine (2), **characterized in** that the device (64) comprises a measuring unit (74) for determining at least one parameter related to the refractive index of the liquid anaesthetic (72), means (92, 94) for determining the temperature of the liquid anaesthetic (72) and an analysis unit (80) for identifying the anaesthetic agent (72) from the determined parameter.

2. A device (64) according to claim 1, **characterized in** that the analysis unit (80) is adapted to establish, from the determined parameter, whether a mixture of at least two liquid anaesthetics is present, and preferably determine the identity of the mixed anaesthetic agents and the composition of the mixture.

3. A device (64) according to claim 1 or 2, **characterized in** that the means (92, 94) for determining the temperature of the liquid anaesthetic (72) comprises a thermometer (92) for measuring the temperature of the liquid anaesthetic (72).

4. A device (64) according to claim 1 or 2, **characterized in** that the means (92, 94) for determining the temperature of the liquid anaesthetic (72) comprises a thermostat (94) for maintaining the temperature of the liquid anaesthetic at a pre-selected temperature.

5. A device (64) according to any of the above claims, **characterized in** that the measuring unit (74) comprises a light-emitting means (76), arranged so a light beam emitted by it strikes a surface of the liquid anaesthetic (72) within a container (70) at a specific angle of incidence ($\alpha$), and a position detector (78) is arranged to indicate the light beam's deflection (x) in the liquid, said deflection (x) constituting the parameter and dependent on the refractive index and the level of the liquid anaesthetic (72).

6. A device (64) according to any of claims 5, **characterized in** that a measuring rod (78) is arranged on the position detector (78) to indicate the identity of the anaesthetic agent (72) when the light beam strikes the position detector (78).

7. A device (64) according to any of the claims 1-4, **characterized in** that the measuring unit (76) also comprises an optical fibre (82) which has a first end arranged by the light-emitting means (76), its other end arranged by a photosensitive means (86) for detecting the parameter and a curved section immersed in the liquid anaesthetic (72), and the measuring unit is arranged to determine the amount of light leaking out of the curved part of the optical fibre as the parameter.

8. A device (64) according to claim 7, **characterized**

**in** that measuring unit (74) also comprises a second optical fibre (84), not immersed in the liquid anaesthetic (72), with the same length as the first fibre (82), said second fibre (84) being intended to supply a reference value, whereupon the analysis unit (80), with the aid of the parameter and reference value, identifies the anaesthetic agent (72) or determines the temperature of the liquid anaesthetic (72) and/or determines the level of the liquid anaesthetic (72).

9. A device (64) according to claim 7 or 8, **characterized in** that the measuring unit (74) also comprises n similarly devised optical fibres ($82_1$, ..., $82n$) arrayed at different levels in the liquid in order to determine the level of the liquid in discrete steps.

10. A device (64) according to claim 7 or 8, **characterized in** that at least one optical fibre (82) has a plurality of curved sections arrayed at different levels in the liquid for determining the level of the liquid.

11. A device (64) according to any of the claims 7 - 10, **characterized in** that the analysis unit (80) issues an alarm if the level of the liquid drops below the curved section of the fibre (82).

12. A device (64) according to claim 7 or 8, **characterized in** that the device (64) also comprises two electrode means (88, 90) arranged to contact with any liquid anaesthetic (72) present in order to measure the capacitance C of liquid between the electrode means (88, 90), the liquid's C varying with and therefore constituting a measure of the level of the liquid.

13. A device (64) according to claim 12, **characterized in** that the immersed part of the optical fibre (82) has two essentially parallel sections, and the two electrode means (88. 90) are arranged along these essentially parallel sections.

14. A device (64) according to claim 13, **characterized in** that the two electrode means (88, 90) consist of a metallized surface area on the parallel parts of the optical fibre (82).

15. A device (64) according to claim 13, **characterized in** that the two electrode means (88, 90) consist of metal wire wound around the optical fibre's (82) parallel sections.

16. A device (64) according to claim 13, **characterized in** that the first electrode means (88) consists of a metallized surface area on the optical fibre (82), and the second electrode means (90) consists of a metal deposit on the inside of a container (70) for the liquid anaesthetic (73).

FIG. 1

FIG. 2

FIG. 3

FIG. 4

FIG. 5

EP 0 823 627 A1

FIG. 6

FIG. 7

FIG. 8

FIG. 9

FIG. 10

FIG. 11

**FIG. 12**

EP 0 823 627 A1

**European Patent Office**

# EUROPEAN SEARCH REPORT

**Application Number**

EP 97 10 9874.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.6) |
|---|---|---|---|
| A | US 4187025 A (ALAN HARMER), 5 February 1980 (05.02.80) | 1-16 | G01N 21/41 |
| A | EP 0483725 A2 (MITSUBISHI DENKI KABUSHIKI KAISHA), 6 May 1992 (06.05.92) | 1-16 | |
| A | US 4834533 A (MASANORI HORIKE ET AL), 30 May 1989 (30.05.89) | 1-16 | |
| A | DE 3400717 A1 (JERUSALEM COLLEGE OF TECHNOLOGY), 19 July 1984 (19.07.84) | 1-16 | |
| A | US 4870292 A (MARTIN D. ALPERT ET AL), 26 September 1989 (26.09.89) | 1-16 | **TECHNICAL FIELDS SEARCHED (Int. Cl.6)** G01N |
| A | EP 0089098 A2 (BATTELLE MEMORIAL INSTITUTE), 21 September 1983 (21.09.83) | 1-16 | |
| A | US 4433913 A (ALAN L. HARMER), 28 February 1984 (28.02.84) | 1-16 | |

The present search report has been drawn up for all claims

| Place of search | Date of completion of the search | Examiner |
|---|---|---|
| STOCKHOLM | 17 November 1997 | JOHANN RIGLER |

CATEGORY OF CITED DOCUMENTS

X : particularly relevant if taken alone
Y : particularly relevant if combined with another document of the same category
A : technological background
O : non-written disclosure
P : intermidiate document

T : theory or principle underlying the invention
E : earlier patent document, but published on, or after the filing date
D : document cited in the application
L : document cited for other reasons
................................................
& : member of the same patent family, corresponding document

European Patent Office

**EUROPEAN SEARCH REPORT**

Application Number
EP 97 10 9874.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.6) |
|---|---|---|---|
| A | US 5396325 A (EDWARD F. CAROME ET AL), 7 March 1995 (07.03.95) ----- | 1-16 | |
| | | | TECHNICAL FIELDS SEARCHED (Int. Cl.6) |

The present search report has been drawn up for all claims

| Place of search | Date of completion of the search | Examiner |
|---|---|---|
| STOCKHOLM | 17 November 1997 | JOHANN RIGLER |

CATEGORY OF CITED DOCUMENTS

X : particularly relevant if taken alone
Y : particularly relevant if combined with another document of the same category
A : technological background
O : non-written disclosure
P : intermidiate document

T : theory or principle underlying the invention
E : earlier patent document, but published on, or after the filing date
D : document cited in the application
L : document cited for other reasons
.............................................
& : member of the same patent family, corresponding document

EPO FORM 1503 03.82 (P0401)